# EUROPEAN PATENT SPECIFICATION

(11) **EP 2 579 880 B1**
(45) Date of publication and mention of the grant of the patent: **11.09.2019**
(21) Application number: 11726105.7
(22) Date of filing: 09.06.2011
(51) Int. Cl.: A61K 31/53, A61K 9/20, A61P 3/10

(54) **PREVENTION OF TYPE 2 DIABETES**
VORBEUGUNG VON TYP-2-DIABETES
PRÉVENTION DU DIABÈTE DE TYPE 2

(30) Priority: 09.06.2010 EP 10305612
(43) Date of publication of application: 17.04.2013
(73) Proprietor: Poxel, 69007 Lyon (FR)
(72) Inventor: FOUQUERAY, Pascale, CH-5643 Sins (CH); CRAVO, Daniel, F-78360 Montesson (FR); HALLAKOU-BOZEC, Sophie, F-92120 Montrouge (FR); BOLZE, Sébastien, F-01600 Massieux (FR)
(74) Representative: Tezier Herman, Béatrice
(86) International application number: PCT/EP2011/059590
(87) International publication number: WO 2011/154497

(56) References cited:
- WO-A1-01/55122
- WO-A2-2004/089917
- WO-A2-2009/028891
- FR-A1- 2 896 159
- WILLIAM KNOWLER ET AL: "Reduction in the Incidence of Type 2 Diabetes with Lifestyle Intervention or Metformin", NEW ENGLAND JOURNAL OF MEDICINE, vol. 346, no. 6, 7 February 2002 (2002-02-07), pages 393-403, XP055111414, ISSN: 0028-4793, DOI: 10.1056/NEJMoa012512
- Anonymous: "Causes of Diabetes", National Diabetes Information Clearinghouse, 1 June 2014 (2014-06-01), XP055270177, Retrieved from the Internet: URL:http://www.niddk.nih.gov/health-inform ation/health-topics/Diabetes/causes-diabet es/Documents/Causes_of_Diabetes_508.pdf [retrieved on 2016-05-03]
- Piero Marchetti ET AL: "Pancreatic Islets from Type 2 Diabetic Patients Have Functional Defects and Increased Apoptosis That Are Ameliorated by Metformin", Journal of Clinical Endocrinology & Metabolism, vol. 89, no. 11, 1 November 2004 (2004-11-01), pages 5535-5541, XP055111413, ISSN: 0021-972X, DOI: 10.1210/jc.2004-0150

## Description

The present invention relates to triazine derivatives for their use in the treatment of type 2 diabetes mellitus, to delay the onset of type 2 diabetes.

### BACKGROUND

Diabetes mellitus is a chronic metabolic disorder of multiple aetiology, characterized by chronic hyperglycemia with disturbance of carbohydrate, fat and protein metabolism resulting from defects in insulin secretion, insulin action, or both. The effect of diabetes mellitus includes long-term damage, dysfunction and failure of various organs. Diabetes mellitus is usually divided into two major categories:
- Type 1 diabetes, (formerly insulin-dependent diabetes mellitus) encompasses the majority of cases which are primarily due to pancreatic islet beta-cell destruction, usually develop in childhood or adolescence and are prone to ketosis and acidosis. Type 1 diabetes accounts for around 10% of all diabetes.
- Type 2 diabetes (formerly non insulin-dependent diabetes mellitus) includes the common major form of diabetes which results from defect(s) in insulin secretion, almost always with a major contribution from insulin resistance. Type 2 diabetes accounts for around 90 % of all diabetes.

The primary progenitor of type 2 diabetes is now presumed to be progressive beta cell dysfunction, which appears early in the clinical course (perhaps antedating and even contributing to the development of insulin resistance) and progressively worsens even under treatment. The physiology of glucose homeostasis requires the close cooperation of a number of organ systems, humoral secretions, and neural signaling complexes; disruption of any of these processes may lead to the development of type 2 diabetes. Predisposing risk factors for type 2 diabetes include overweight and obesity, poor diet, and lack of exercise. Genetic factors, many of which as yet require elucidation, may also elevate the risk of developing type 2 diabetes. Insulin resistance has long been recognized as a primary, if not the primary, cause of type 2 diabetes. Recent research in disease pathogenesis suggests that insulin resistance is neither a necessary nor a sufficient condition for development and progression of type 2 diabetes. Although insulin resistance is highly correlated with type 2 diabetes, many individuals with insulin resistance will not go on to develop the disease; moreover the disease may be present in individuals not markedly insulin resistant. Among the mechanisms of beta cell dysfunction in type 2 diabetes is the reduction or abrogation of the "incretin effect". The incretins are gut hormones, glucagon-like peptide-1 (GLP-1) and glucose-dependent insulinotropic polypeptide (GIP), which in healthy individuals potentiate glucose-dependent insulin secretion. In addition, these hormones, and particularly GLP-1, have a number of protective effects on the beta cell, including reduction in apoptosis and promotion of beta cell proliferation and neogenesis.

As these benefits are lost in diabetes, "repairing" the incretin effect has become an important treatment target. Treatments that maintain the beta cell could offer durable glycemic control and potentially reduce the micro- and macrovascular complications associated with type 2 diabetes (Campbell RK. Fate of the beta cell in the pathophysiology of type 2 diabetes. J Am Pharm Assoc (2003). 2009 Sep-Oct;49 Suppl 1:S10-5).

In type 2 diabetes, the beta cells of the pancreas fail to produce enough insulin to meet the body's demand, in part because of an acquired decrease in beta cell mass. In adults, pancreatic beta cell mass is controlled by several mechanisms, including beta cell replication, neogenesis, hypertrophy, and survival. It appears that increased beta cell apoptosis is an important factor contributing to beta cell loss and the onset of type 2 diabetes (Rhodes CJ. Type 2 diabetes-a matter of beta cell life and death, Science, 2005, Jan 21; 307(5708):380-4). In early stages of type 2 diabetes, pancreatic beta cells adapt to insulin resistance by increasing mass and function. As nutrient excess persists, hyperglycemia and elevated free fatty acids negatively impact beta cell function. This happens by numerous mechanisms, including the generation of reactive oxygen species, alterations in metabolic pathways, increases in intracellular calcium and the activation of endoplasmic reticulum stress. These processes adversely affect beta cells by impairing insulin secretion, decreasing insulin gene expression and ultimately causing apoptosis. Pancreatic beta cells possess the potential to greatly expand their function and mass in both physiologic and pathologic states of nutrient excess and increased insulin demand. Beta-cell response to nutrient excess occurs by several mechanisms, including hypertrophy and proliferation of existing beta cells, increased insulin production and secretion, and formation of new beta cells from progenitor cells. Failure of pancreatic beta cells to adequately expand in settings of increased insulin demand results in hyperglycemia and diabetes (Chang-Chen KJ, Mullur R, Bernal-Mizrachi E., Beta-cell failure as a complication of diabetes, Rev Endocr Metab Disord, 2008 Dec.9(4):329-43).

Type 2 diabetes can affect many major organs, including heart, blood vessels, nerves, eyes and kidneys leading to various diseases, including cardiovascular diseases, neuropathy, ulcers, retinopathy and nephropathy. Treatments that maintain the beta cell could offer durable glycemic control and potentially reduce complications associated with type 2 diabetes.

WO 01/055122, WO 2004/089917 and FR 2 896 159 describe the administration of triazine compounds, including (+)-2-amino-3,6-dihydro-4-dimethylamino-6-methyl-1,3,5-triazine hydrochloride, to diabetic humans (or rats in the examples), which differ from pre-diabetic patients by their plasma glucose level. Other structurally different compounds have been disclosed for treating type 2 diabetes, such as WO 2009/028891 which discloses 1,3,5-triazine-2,4,6-triamine compounds. However, it is not suggested to administer these compounds to pre-diabetic patients. In addition, it has been known to use metformin in the prevention (W. Knowler et al., New England Journal of Medicine, vol. 346, No. 6, 393-403 (2002)) or treatment (P. Marchetti et al., Journal of Clinical Endocrinology & Metabolism, vol.89, No. 11, 5535-5541 (2004)) of diabetes.

### SUMMARY OF THE INVENTION

In view of the above, it would be advantageous to have a type 2 diabetes treatment which could maintain the beta cell function or prevent beta cell dysfunction. In particular, it would be especially advantageous to have a treatment able to delay the onset of type 2 diabetes, especially at early stages, in particular where beta cells dysfunction has not reached the critical point yet.

The inventors have unexpectedly discovered that a specific triazine derivative was efficient at protecting beta cells from cellular death, and could consequently be useful in the treatment of type 2 diabetes by delaying the onset of type 2 diabetes. This would be an help to prevent or delay beta cell dysfunction in at risk population before the onset of type 2 diabetes, in particular for subjects having pre-diabetes.

International patent application WO01/055122 describes triazine derivatives and their hypoglycaemic properties. To our knowledge, the activity of such triazine derivatives in protecting beta cells from destruction or dysfunction and their use in the treatment to delay the onset of type 2 diabetes have never been described.

### BRIEF DESCRIPTION OF THE FIGURES

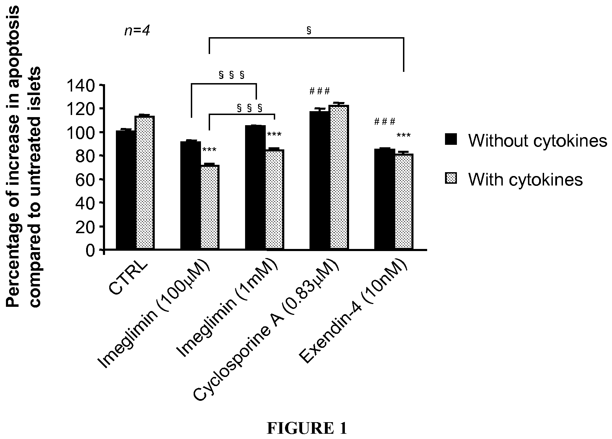

Figure 1: Percentage of increase in apoptosis compared to untreated rat islets (set to 100%). Values represent the mean+/-sem of four independent experiments in duplicate. Islets from six rats were pooled for each experiment. §P<0.05 and §§§P<0.001. ### P<0.001 when specifically compared to control without cytokines. ***P<0.001 when specifically compared to control with cytokines.

### DETAILED DESCRIPTION OF THE INVENTION

The first object of the present invention is a triazine derivative chosen from (+)-2-amino-3,6-dihydro-4-dimethylamino-6-methyl-1,3,5-triazine or a pharmaceutically acceptable salt thereof, for use in a method for the treatment to delay the onset of type 2 diabetes by protecting beta cells from cellular death, wherein the subject to be treated has pre-diabetes.

In a preferred embodiment, the triazine derivative is (+)-2-amino-3,6-dihydro-4-dimethylamino-6-methyl-1,3,5-triazine hydrochloride.

Disclosed herein are prodrugs of the compounds defined above.

The term "prodrugs" means compounds which, when administered to the patient, are chemically and/or biologically converted in the live body into (+)-2-amino-3,6-dihydro-4-dimethylamino-6-methyl-1,3,5-triazine .

The process for the preparation of the compounds according to the invention is especially described in patent application WO 2004/089917.

The present disclosure also concerns the polymorphic forms of the compounds, as obtained according to patent application WO 2004/089917, for instance the A1 or HI polymorphic form of (+)-2-amino-3,6-dihydro-4-dimethylamino-6-methyl-1,3,5-triazine hydrochloride salts.

Triazine derivatives have been shown to be efficient at protecting beta cells from cellular death.

The triazine derivatives as defined above may be used in the preparation of a medicament for the treatment of type 2 diabetes, in particular to delay the onset of type 2 diabetes.

Reference is also made herein to a pharmaceutical composition comprising at least one triazine derivative as defined above and a pharmaceutically acceptable support. The pharmaceutical composition comprising the triazine derivative as defined above can be prepared by mixing the triazine derivative with a physiologically acceptable support, an excipient, a binder or a diluent.

Reference is also made herein to the pharmaceutical composition as defined above for its use in the treatment to delay the onset of type 2 diabetes, and to a method of treatment to delay the onset of type 2 diabetes, comprising administration to a subject in need thereof of an effective amount of a triazine derivative as defined above or of a pharmaceutical composition comprising an effective amount of a triazine derivative as defined above.

The derivatives of the invention can be administered to those who do not have all symptoms of type 2 diabetes but who would normally develop the type 2 diabetes or be at increased risk for type 2 diabetes. Treatment includes delaying the development of the disease in an individual who will ultimately develop all symptoms of type 2 diabetes or would be at risk for the disease due to age, familial history, genetic or chromosomal abnormalities, and/or due to the presence of one or more biological markers for the disease. By delaying the onset of the disease, derivatives of the invention prevent the individual from getting the disease during the period in which the individual would normally have gotten the disease or reduce the rate of development of the disease or some of its effects. Treatment also includes administration of the derivatives of the invention to those individuals thought to be predisposed to type 2 diabetes. In treating type 2 diabetes, the derivatives of the invention are administered in a therapeutically effective amount.

As mentioned before, type 2 diabetes can affect many major organs, including heart, blood vessels, nerves, eyes and kidneys. Complications associated with type 2 diabetes can correspond to various diseases, such as for instance cardiovascular diseases, neuropathy, ulcers (i.e., foot ulcers), retinopathy or nephropathy.

Cardiovascular diseases include more particularly high blood pressure, coronary artery disease, heart disease and/or stroke.

The medicament for treating type 2 diabetes comprising a triazine derivative according to the invention is administrated to a subject in need thereof.

Subjects in need of such treatment may be diagnosed by implementing the following tests:
- A fasting plasma glucose (FPG) test measures blood glucose in a person who has not eaten anything for several hours, such as for at least 8 hours. This test is used to detect diabetes and pre-diabetes.
- An oral glucose tolerance test (OGTT) measures blood glucose after a person fasts at least 8 hours and 2 hours after the person drinks a glucose-containing beverage. This test can be used to diagnose diabetes and pre-diabetes.
- A random plasma glucose test, also called a casual plasma glucose test, measures blood glucose without regard to when the person being tested last ate. This test, along with an assessment of symptoms, is used to diagnose diabetes but not pre-diabetes.

Test results indicating that a subject has diabetes could be confirmed with a second test on a different day.

Depending on the obtained test results, subjects can be diagnosed as being normal, pre-diabetes or diabetes subjects. Pre-diabetes precedes the onset of type 2 diabetes. Generally, subjects who have pre-diabetes have fasting blood glucose levels that are higher than normal, but not yet high enough to be classified as diabetes. Pre-diabetes greatly increases the risk for diabetes.

One aim of the treatment of the invention is in these groups of patients to delay the onset or progression of type 2 diabetes.

In the present invention, subjects in need of the treatment of the invention are subjects having pre-diabetes.

The amount of triazine derivative as defined above to be administered may evolve in a large scope depending upon the patient, the mode of administration and the expected effect. In particular, the amount of triazine derivative to be administered is comprised between 200 mg and 4000 mg, preferably between 500 and 3000 mg, in particular between 1000 and 2000 mg, per day.

The derivative according to the invention can be administered orally or non-orally, for instance via parenteral, intravenous, cutaneous, nasal or rectal route.

The pharmaceutical composition can present different forms including granules, powders, tablets, gel capsules, syrups, emulsions, suspensions, and forms used for non-oral administration, for instance injections, sprays or suppositories. These pharmaceutical forms can be prepared via known conventional techniques.

The preparation of an orally administered solid pharmaceutical form can be for instance performed by the following process: an excipient (for example lactose, sucrose, starch or mannitol), a disintegrant (for example calcium carbonate, calcium carboxymethylcellulose, alginic acid, sodium carboxymethylcellulose, colloidal silicon dioxide, sodium croscarmellose, Crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose, polyoxyethylene glycol, cellulose acetophthalate, hydroxypropylmethylcellulose phthalate and Eudragit® (methacrylic acid-acrylic acid copolymer), Opadry® (hydroxypropylmethylcellulose + macrogol + titanium oxide + lactose monohydrate). Pharmaceutically acceptable colorants may be added (for example yellow iron oxide, red iron oxide or quinoline yellow lake).

Liquid pharmaceutical forms for oral administration include solutions, suspensions and emulsions. The aqueous solutions can be obtained by dissolving the active principle in water, followed by addition of flavourings, colorants, stabilisers and/or thickeners, if necessary. In order to improve the solubility, it is possible to add ethanol, propylene glycol or any other pharmaceutically acceptable non-aqueous solvent. The aqueous suspensions for oral use can be obtained by dispersing the finely divided active principle in water with a viscous product, such as a natural or synthetic gum or resin, methylcellulose or sodium carboxymethylcellulose.

The pharmaceutical forms for injection can be obtained, for example, by the following process. The active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween 80, HCO 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilising agent (for example sodium salicylate or sodium acetate) or a stabiliser (for example human serum albumin).

Pharmaceutical forms for external use can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

In an embodiment, the triazine derivative is the only active principle used according to the invention or is the only active principle in the composition.

The triazine derivative according to the invention may be co-administered with at least one other active compound. Preferably, the at least one other active compound is chosen among treatments currently used to treat pre-diabetes or type 2 diabetes. The term "co-administration" (or "co-administrered") means the simultaneous, separate or sequential administration of one or more compounds to the same patient, over a period that may be up to 2 hours or even up to 12 hours. For example, the term co-administration includes (1) a simultaneous administration of both compounds, (2) an administration of the first, followed 2 hours later by the administration of the second compound, (3) an administration of the first, followed 12 hours later by the administration of the second compound.

The examples below are given as non-limiting illustrations of the invention.

### EXAMPLES

### Example 1: Study of the protective effect of a triazine derivative on beta-cells death

Rat pancreatic islets were cultured for 24 hours with or without a cocktail of cytokines (TNF-alpha, IL-lbeta and INF-gamma each at 2 ng/ml), creating inflammatory stress, in order to quantify the protective properties of (+)-2-amino-3,6-dihydro-4-dimethylamino-6-methyl-1,3,5-triazine hydrochloride . Both doses of (+)-2-amino-3,6-dihydro-4-dimethylamino-6-methyl-1,3,5-triazine hydrochloride completely protected the islets from the cytokine stress.

These results allow concluding that (+)-2-amino-3,6-dihydro-4-dimethylamino-6-methyl-1,3,5-triazine hydrochloride has protective properties against basal and inflammation-like apoptosis on cultured rat islets.

### Experimental procedure:

Male Wistar rat islets (Elevage Janvier, Le Genest-St-Isle, France) were isolated using collagenase (Serva, Heidelberg, Germany) and cultured in RPMI at 11mM glucose (Invitrogen, CA, USA) supplemented with 10% FCS (Fetal Calf Serum), 100 Units/ml penicillin, 100 µg/ml streptomycin and 100 µg/ml gentamycin in low-attachment 24-well plates (Corning, NY, USA). These islets were incubated with a cocktail of rat cytokines (TNF-alpha, IL-lbeta and INF-gamma each at 2 ng/ml) for 24 hours (Brun T. *et al.,* 2004 and 2007). 1 hour prior to cytokine incubation, (+)-2-amino-3,6-dihydro-4-dimethylamino-6-methyl-1,3,5-triazine hydrochloride (Imeglimin) at 100 µM or 1 mM, Cyclosporine A (CsA) at 0.83 µM or Exendin-4 (Exe-4) at 10 nM (Sigma, MO, USA) was added separately to the medium to measure the protective effect of each drug on stressed or unstressed islets. Aliquots of the medium were used to measure insulin accumulated during the culture period using immune enzyme assay kits (Brunchwig, Basel, Switzerland). Furthermore, quantification of cytoplasmic nucleosomes, a direct indicator of apoptosis, was performed with the Cell Death Detection ELISA kit (Roche, Basel, Switzerland). Each condition was tested with 50 rat islets in duplicate and four independent experiments were performed. The results are provided as mean+/-sem and statistical analyses were performed using one-way ANOVA with Bonferroni and Dunnett post-hoc tests. The different conditions are summarized in the table below.

| **Condition #** | **1** | **2** | **3** | **4** | **5** |
|---|---|---|---|---|---|
| Treatment of Islets | non-treated - cytokines | 100 µM Imeglimin - cytokines | 1 mM Imeglimin - cytokines | CsA 0.83 µM - cytokines | Exe-4 10nM - cytokines |
| Nbr of Islets per duplicate | 50 | 50 | 50 | 50 | 50 |

| **Condition #** | **6** | **7** | **8** | **9** | **10** |
|---|---|---|---|---|---|
| Treatment of Islets | non-treated + cytokines | 100 µM Imeglimin + cytokines | 1 mM Imeglimin + cytokines | CsA 0.83 µM + cytokines | Exe-4 10 nM + cytokines |
| Nbr of Islets per duplicate | 50 | 50 | 50 | 50 | 50 |

### Results:

To quantify the protective effect of (+)-2-amino-3,6-dihydro-4-dimethylamino-6-methyl-1,3,5-triazine hydrochloride on rat pancreatic islets, two doses of the compound (100 µM and 1 mM) were added to the medium 1-hour prior to a 24-hour incubation with or without a cocktail of rat cytokines (TNF-alpha, IL-lbeta and INF-gamma). The conditions without cytokines allowed the analysis of the specific effect of the compound on islets while the combination of the drug with cytokines allowed the study of the effect of Imeglimin on islets under an inflammatory stress. To measure cell death in islets, quantification of cytoplasmic nucleosomes in islet cells, a consequence of DNA fragmentation during apoptosis (Robertson *et al.,* 2000), was performed. To be able to compare the results with a double reference, the level of cell death in untreated islets but also in islets incubated only with cytokines was analyzed. Furthermore, a negative control and a positive control were added to the conditions with and without cytokines to test the effectiveness of the protocol. Cyclosporine A, reported to be toxic in pancreatic islets (Hahn *et al.,* 1986) but protective in other cell types (Tharakan B. *et al.,* 2009; Fauvel H. *et al.,* 2002; Sullivan P.G. *et al.,* 2000), was selected as the negative control while Exendin-4, with potent protective properties against cell death (Li *et al.,* 2003; Wang and Brubaker, 2002), was used as the internal positive control. The results of the quantification of cell death for each condition are presented on figure 1.

For conditions without cytokine addition, (+)-2-amino-3,6-dihydro-4-dimethylamino-6-methyl-1,3,5-triazine hydrochloride at 100 µM had protective effects (10% less apoptosis than untreated islets). Cyclosporine A by itself induced a 17% increase in apoptosis. Exendin-4 showed the opposite effect with a 16% decrease in cell death. These results indicate that (+)-2-amino-3,6-dihydro-4-dimethylamino-6-methyl-1,3,5-triazine hydrochloride is by itself protective with a level of protection representing two thirds of the Exendin-4 level.

Cytokine treatment caused a 12% increase in cell death compared to untreated islets. In the presence of cytokines, (+)-2-amino-3,6-dihydro-4-dimethylamino-6-methyl-1,3,5-triazine hydrochloride at 100 µM had an even greater protective effect (37% less apoptosis than cytokine-stressed islets or 29% less apoptosis than untreated islets) while (+)-2-amino-3,6-dihydro-4-dimethylamino-6-methyl-1,3,5-triazine hydrochloride at ImM protected by 25% or 16% respectively. Cyclosporine A induced an 8% or 22% increase respectively, and Exendin-4 a drop of 29% or 20% in apoptosis. These values show a protective effect of (+)-2-amino-3,6-dihydro-4-dimethylamino-6-methyl-1,3,5-triazine hydrochloride. The lower concentration tested is nearly 50% more potent than Exendin-4.

### Example 2: Compositions according to the invention

### Formulation 1:

(+)-2-amino-3,6-dihydro-4-dimethylamino-6-methyl-1,3,5-triazine hydrochloride: 1000 mg
microcrystalline cellulose: 114 mg
croscarmellose: 30 mg
polyvinylpyrrolidone: 40 mg
magnesium stearate: 15 mg
Opadry®: 25 mg

### Formulation 2:

(+)-2-amino-3,6-dihydro-4-dimethylamino-6-methyl-1,3,5-triazine hydrochloride: 750 mg
microcrystalline cellulose: 110 mg
croscarmellose: 21 mg
polyvinylpyrrolidone: 30 mg
magnesium stearate: 10.5 mg
Opadry®: 20 mg

### Formulation 3:

(+)-2-amino-3,6-dihydro-4-dimethylamino-6-methyl-1,3,5-triazine hydrochloride: 1000 mg
microcrystalline cellulose: 150 mg
croscarmellose: 25 mg
polyvinylpyrrolidone: 45 mg
magnesium stearate: 10mg
Eudragit®: 25 mg.

## Claims

1. Triazine derivative chosen from (+)-2-amino-3,6-dihydro-4-dimethylamino-6-methyl-1,3,5-triazine or a pharmaceutically acceptable salt thereof, for use in a method for the treatment to delay the onset of type 2 diabetes by protecting beta cells from cellular death, wherein the subject to be treated has pre-diabetes.

2. Triazine derivative for a use according to claim 1, wherein the pharmaceutically acceptable salt is hydrochloride.

## Patentansprüche

1. Triazinderivat ausgewählt aus (+)2-Amino-3,6-dihydro-4-dimethylamino-6-methyl-l,3,5-triazin oder einem pharmazeutisch unbedenklichen Salz davon, zur Verwendung in einem Behandlungsverfahren zum Verzögern des Einsetzens von Typ-2-Diabetes durch Schützen von Betazellen vor dem Zelltod, wobei das behandelte Subjekt an Prädiabetes leidet.

2. Triazinderivat zur Verwendung nach Anspruch 1, wobei es sich bei dem pharmazeutisch unbedenklichen Salz um Hydrochlorid handelt.

## Revendications

1. Dérivé de triazine choisi parmi la (+)-2-amino-3,6-dihydro-4-diméthylamino-6-méthyl-l,3,5-triazine ou un sel pharmaceutiquement acceptable, pour une utilisation dans une méthode de traitement destinée à retarder l'apparition du diabète de type 2 en protégeant les cellules bêta contre la mort cellulaire, le sujet à traiter ayant un prédiabète.

2. Dérivé de triazine selon la revendication 1, le sel pharmaceutiquement acceptable étant un chlorhydrate.
